# EUROPÄISCHE PATENTSCHRIFT

(11) **EP 2 739 602 B1**
(45) Veröffentlichungstag und Bekanntmachung des Hinweises auf die Patenterteilung: **30.08.2017**
(21) Anmeldenummer: 12743139.3
(22) Anmeldetag: 01.08.2012
(51) Int. Cl.: C07C 209/08

(54) **VERFAHREN ZUR HERSTELLUNG VON KERNHALOGENIERTEN N,N-DIALKYLBENZYLAMINEN**
METHOD FOR PRODUCING RING-HALOGENATED N,N-DIALKYLBENZYLAMINES
PROCÉDÉ DE PRÉPARATION DE N,N-DIALKYLBENZYLAMINES À HALOGÉNATION SUR LE NOYAU

(30) Priorität: 04.08.2011 EP 11176506
(43) Veröffentlichungstag der Anmeldung: 11.06.2014
(73) Patentinhaber: LANXESS Deutschland GmbH, 50569 Köln (DE)
(72) Erfinder: HARTWIG, Jordan, 51467 Bergisch Gladbach (DE)
(86) Internationale Anmeldenummer: PCT/EP2012/064993
(87) Internationale Veröffentlichungsnummer: WO 2013/017611

(56) Entgegenhaltungen:
- CHINGAKHAM B. SINGH ET AL: "Aqueous-MediatedN-Alkylation of Amines", EUROPEAN JOURNAL OF ORGANIC CHEMISTRY, Bd. 2007, Nr. 8, 1. März 2007 (2007-03-01) , Seiten 1369-1377, XP55039887, ISSN: 1434-193X, DOI: 10.1002/ejoc.200600937
- JULIUS V. BRAUN, MARTIN KÜHN UND JOSEF WEISMANTEL: "Haftfestigkeit organischer Reste", LIEBIGS ANNALEN DER CHEMIE, Bd. 449, 1926, Seiten 249-277, XP002684509, in der Anmeldung erwähnt

## Beschreibung

Die Erfindung betrifft ein Verfahren zur Herstellung von kernhalogenierten N,N-Dialkyl-benzylaminen und daraus erhältlichen Zwischenstufen für die Herstellung von Agrochemikalien und pharmazeutischen Wirkstoffen.

Kernhalogenierte N,N-Dialkylbenzylamine stellen wertvolle Zwischenprodukte in Verfahren zur Herstellung von Agrochemikalien und pharmazeutischen Wirkstoffen dar, da sie durch Metallierung wie beispielsweise Lithiierung oder Umsetzung zu Grignardreagenzien eine weitere Substitution am aromatischen Ring zum Beispiel durch Umsetzung mit Oxalsäureestern erlauben.

So ist beispielsweise in JP 2003 026640 A beschrieben, 2-Chlor-N,N-dialkylbenzylamine mit Magnesium und Ethylbromid in Toluol und THF zunächst zu 2-(Butoxycarbonylcarbonyl)-N,N-dialkylbenzylaminen umzusetzen, die selbst wiederum Verwendung zur Herstellung von gewissen Fungiziden des Strobilurintyps wie zum Beispiel Kresoxim-methyl dienen können.

Die Herstellung der als Ausgangsprodukt verwendeten kernhalogenierte N,N-Dialkylbenzylamine ist grundsätzlich bekannt. S. Bhattacharyya, Synth. Commun. 2000, 30, 2001-2008 beschreibt die Umsetzung von 2-Chlorbenzaldehyd mit Dimethylamin zum entsprechenden Iminiumsalz sowie die nachfolgende Reduktion mit Natriumborhydrid zum Zielprodukt.

Eine Leuckart-Wallach-Reaktion von 2-Chlorbenzylamin mit Formaldehyd und Ameisensäure ist in S. H. Pine et al., J. Org. Chem., 1971, 36, 984-991 beschrieben.

Braun et al. beschreiben in Liebigs Ann. Chem. 1926, 449, 249-277 die Umsetzung von o-Chlorbenzylchlorid mit Dimethylamin im geschlossenen Rohr bei 100°C. Es werden keine Angaben zum Verhältnis der beiden Komponenten und zur Aufarbeitung gemacht. Der Nachteil dieses Verfahrens besteht jedoch darin, dass das als Nebenprodukt anfallende Dimethylamin-Hydrochlorid kristallin anfällt und umständlich vom 2-Chlor-N,N-dimethylbenzylamin abgetrennt werden muss.

Ein weiteres Verfahren wird bei J. H. Short et al., J. Pharm. Sci. 1962, 51, 881-884 beschrieben, wo auf die Synthesemethode von E. L. Eliel et al. (J. Org. Chem. 1954, 19, 1693-1698) verwiesen wird. Dabei wird Benzol mit gasförmigem Dimethylamin gesättigt und bei Raumtemperatur mit o-Chlorbenzylchlorid drucklos umgesetzt. Abgesehen vom Aufwand einer erforderlichen Lösemittelabtrennung hat dieses Verfahren den entscheidenden Nachteil, dass durch die drucklose Fahrweise nur ungünstige Eduktverhältnisse realisiert werden können, die insbesondere bei der niedrigen Reaktionstemperatur die Bildung von unerwünschten quarternären Ammoniumsalzen fördert.

In DE 676331 wird vorgeschlagen o-Chlorbenzylchlorid mit Dimethylamin umzusetzen und das anfallende Dimethylamin-Hydrochlorid bei hohen Temperaturen als Schmelze abzutrennen.

Singh et al. beschreiben in Eur. J. Org. Chem. 2007, 1369-1377 ein Verfahren zur Herstellung von Benzylbutyl-phenyl-amin durch Umsetzung von Butyl-phenyl-amin mit Benzylbromid in Wasser bei 80°C in Anwesenheit von Natriumdodecasulfat als Katalysator und NaHCO₃ als stöchiometrischer Base.

All den vorgenannten Syntheseverfahren ist gemeinsam, dass sie unerwünschte Nebenprodukte erzeugen, bei Synthesen ausgehend von Dimethylamin mit o-Chlorbenzylchlorid ist insbesondere der Anfall an o-Chlorbenzaldehyd als Nebenkomponente zu beobachten.

Da diese Komponente einen sehr ähnlichen Siedepunkt wie das Zielprodukt 2-Chlor-N,N-dimethylbenzylamin hat, kann sie durch Destillation nicht wirksam abgetrennt werden und stört insbesondere bei Metallierungsreaktionen durch Folgereaktionen massiv.

Des Weiteren wird insbesondere bei kommerziell verfügbarem 2-Chlorbenzylchlorid, das typischerweise durch radikalische Chlorierung von 2-Chlortoluol gewonnen wird, häufig als Verunreinigung auch 2-Chlor-benzalchlorid beobachtet, das für die Aminierung störend ist.

Es bestand daher der Bedarf an einem Verfahren, das im technischen Maßstab durchführbar ist und hohe Ausbeuten liefert sowie vorzugsweise die Herstellung kernhalogenierter N,N-dialkylbenzylamine mit einem Gehalt von 500 ppm oder weniger bezogen auf das Gewicht an entsprechenden kernhalogenierten Benzaldehyden erlaubt.

Es wurde nun ein Verfahren zur Herstellung von Verbindungen der Formel (I) gefunden 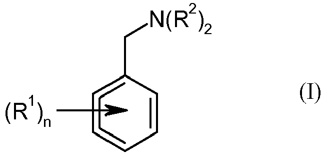 in der
- n: für 0, 1, 2 oder 3, bevorzugt für 1 oder 2, besonders bevorzugt für 1 steht
- die Reste R¹: jeweils unabhängig voneinander für ein Halogenatom stehen, dass an den aromatischen Kern gebunden ist, vorzugsweise für Chlor oder Brom, noch weiter bevorzugt für Chlor
- die Reste R²: jeweils unabhängig voneinander für C₁-C₈-Alkyl, C₆-C₁₂-Aryl oder C₇-C₁₃-Arylalkyl oder die beiden Reste R² zusammen für einen geradkettigen oder verzweigten C₃-C₁₂-Alkylen-Rest stehen, der gegebenenfalls durch ein oder mehrere Sauerstoffatome unterbrochen sein kann
durch Umsetzung von Verbindungen der Formel (II) 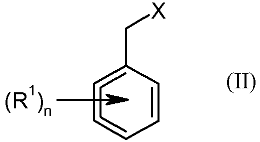 mit Verbindungen der Formel (III)

HN(R²)₂ (III)

wobei in den Formeln (I) und (II) die Reste R¹, und R³ die unter der Formel (I) genannte Bedeutung besitzen und
- X: für Chlor, Brom, Iod, -OSO₂-(C₁-C₆-Alkyl) oder -OSO₂-(C₁-C₆-Perfluoralkyl), bevorzugt für Chlor oder Brom, ganz besonders bevorzugt für Chlor steht
wobei das Verfahren dadurch gekennzeichnet ist, dass
• das molare Verhältnis von Verbindungen der Formel (III) zu Verbindungen der Formel (II) 3:1 oder größer ist, vorzugsweise 3:1 bis 50:1, besonders bevorzugt 3:1 bis 20:1 und ganz besonders bevorzugt 5:1 bis 20:1 sowie in einer anderen Ausführungsform 5:1 oder größer, vorzugsweise 5:1 bis 50:1, besonders bevorzugt 5:1 bis 20:1 und ganz besonders bevorzugt 8:1 bis 14:1
• organische Lösungsmittel in einer Menge von 10 Gew.-% oder weniger, vorzugsweise 5 Gew.-% oder weniger, besonders bevorzugt 0 bis 2 Gew.-% bezogen auf die Summe an eingesetzten Verbindungen der Formel (II) und (III) eingesetzt werden und ganz besonders bevorzugt kein organisches Lösungsmittel eingesetzt wird und
• Wasser eingesetzt wird, vorzugsweise mehr als 10 Gew. % vorzugsweise mehr als 30 Gew.-%, ganz besonders bevorzugt mehr als 50 Gew.-% und insbesondere mehr als 100 Gew.-% Wasser vorzugsweise 100 bis 1000 Gew.-% bezogen auf die eingesetzte Verbindung der Formel (II),
   bei einer Reaktionstemperatur von 90-180°C.
Im Rahmen der Erfindung können die aufgeführten, allgemeinen oder in Vorzugsbereichen aufgeführten Restedefinitionen bzw. Erläuterungen untereinander, also auch zwischen den jeweiligen Bereichen und Vorzugsbereichen beliebig kombiniert werden.

Die beiden Reste in den Verbindungen der Formel (I) und (II)
- R²: stehen bevorzugt jeweils unabhängig voneinander, besonders bevorzugt jeweils identisch für C₁-C₈-Alkyl oder zusammen für einen geradkettigen oder verzweigten C₅-C₆-Alkylen-Rest oder einen Ethandiyl-oxo-Ethandiyl-Rest
ganz besonders bevorzugt jeweils identisch für Methyl, Ethyl, n-Propyl, n-Butyl, 1,4-Butandiyl oder 1,5-Pentandiyl, wobei Methyl und Ethyl weiter und Methyl noch weiter bevorzugt ist.

Für Fälle, in denen n = 1 ist, steht
- der Rest R¹: vorzugsweise für einen zur Benzylgruppe orthoständigen Chlor oder Brom-Rest vorzugsweise für einen orthoständigen Chlor-Rest

Eine besonders bevorzugte Verbindung der Formel (I) ist 2-Chlor-N,N-dimethylbenzylamin.

Eine besonders bevorzugte Verbindung der Formel (II) ist 2-Chlor-benzylchlorid.

Eine besonders bevorzugte Verbindung der Formel (III) ist Dimethylamin.

Die Reaktionstemperatur liegt zwischen 90 und 180°C, ganz besonders bevorzugt 130 bis 150°C.

Der Reaktionsdruck hängt typischerweise stark vom Dampfdruck der eingesetzten Verbindung der Formel (III) und dem Dampfdruck des Wassers bei der Reaktionstemperatur ab und kann beispielsweise 2 bis 300 bar, vorzugsweise 4 bis 50 bar und besonders bevorzugt 5 bis 35 bar betragen. In den Beispielen stellte sich der Druck auf Werte zwischen 8 und 31 bar ein.

Die Reaktionsdauer beträgt typischerweise zwischen 5 min und 24 Stunden vorzugsweise zwischen 30 min und 5 Stunden. Längere Reaktionsdauern sind nicht schädlich, haben aber auch keinen Vorteil mehr.

Die überschüssige Verbindung der Formel (III) kann zur Rückführung in die Reaktion beispielsweise in an sich bekannter Weise durch thermische Behandlung wieder gewonnen werden, idealerweise durch Druckdestillation.

Bei der Reaktion bilden sich auch die entsprechenden Hydrohalogenide der Verbindungen der Formel (III), die zumindest teilweise, vorzugsweise vollständig in Wasser gelöst vorliegen und die ebenfalls in an sich bekannter Weise durch Zusatz von Base freigesetzt und wieder gewonnen werden können. Als Basen eignen sich dafür insbesondere Alkali- und Erdalaklimetallcarbonate und -hydroxide wie beispielsweise Natrium-, Kalium- und Calciumhydroxid oder -carbonat, bevorzugt ist Natriumhydroxid.

Das Verfahren kann sowohl batchweise als auch kontinuierlich durchgeführt werden, wobei die kontinuierliche Verfahrensweise bevorzugt ist.

Für die kontinuierliche Verfahrensweise kann beispielsweise ein Röhrenreaktor eingesetzt werden in den die Einsatzstoffe eingepumpt werden. Nach Durchlaufen der Verweilstrecke wird der Druck des Reaktionsgemisches reduziert und die überschüssige Verbindung der Formel (III) in einer Druckdestillation zurück gewonnen.

Die weitere Aufarbeitung kann dann beispielsweise in einem batch-Prozess erfolgen, wobei die Behandlung mit Base z.B. in einer Mixer-Settler-Apparatur erfolgen kann.

Die wässrige Phase wird von der organischen Phase, die das Produkt enthält bzw. durch das Produkt gebildet wird abgetrennt und die organische Phase dann vorzugsweise durch Destillation gereinigt. Die oben beschriebene Umsetzung mit Base kann vor oder nach Abtrennung der wässrigen Phase erfolgen.

Erfindungsgemäß können die Verbindungen der Formel (I) in hervorragenden Ausbeuten von über 92 % in einigen Fällen von über 95 % der Theorie erhalten werden. Die Reinreiten erreichen nach Destillation bis zu 99,9 Gew.-%.

Überraschend wurde weiterhin gefunden, dass die Verbindungen der Formel (I) von Restgehalten an Benzaldehyden befreit werden können in dem in einem weiteren Schritt

Verbindungen der Formel (I) mit einem Gehalt an Verbindungen der Formel (IV) 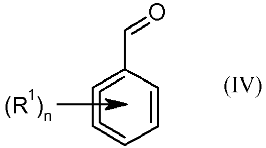 von vorzugsweise 0,050 bis 2,0 Gew.-%, besonders bevorzugt 0,1 bis 1,0 Gew.-% mit einem Alkalimetallborhydrid oder einem Alkalimetallaluminiumhydrid umgesetzt werden und gegebenenfalls aber vorzugsweise anschließend destilliert werden.

Bevorzugte Alkalimetallborhydride sind Lithiumborhydrid und Natriumborhydrid, ein bevorzugtes Alkalimetallaluminiumhydrid ist Lithiumaluminiumhydrid LiAlH₄.

Bevorzugt ist der Zusatz von Natriumborhydrid.

Eine besonders bevorzugte Verbindung der Formel (IV) ist 2-Chlorbenzaldehyd, der bei der Herstellung von 2-Chlor-N,N-dimethylbenzylamin auftritt.

Die eingesetzte Menge kann beispielsweise zwischen 0,01 und 5 Gew.-%, vorzugsweise zwischen 0,02 und 2 Gew-%, besonders bevorzugt zwischen 0,02 und 0,4 Gew.-% bezogen auf das Gewicht der eingesetzten Verbindung der Formel (I) mit einem Gehalt an Verbindung der Formel (IV) betragen.

Die Reaktionstemperatur liegt hierbei beispielsweise bei 0 und 150°, bevorzugt bei 15 und 100°C, ganz besonders bevorzugt bei 40 bis 80°C.

Die Reaktionsdauer beträgt typischerweise zwischen 5 min und 24 Stunden vorzugsweise zwischen 30 min und 5 Stunden.

Längere Reaktionsdauern oder höhere Mengen an Alkalimetallbor- oder -aluminiumhydriden sind nicht schädlich, bringen aber auch keinen Vorteil mehr.

Das Ende der Reaktion kann beispielsweise mittels HPLC verfolgt werden.

Die erfindungsgemäß erhaltenen Verbindungen weisen einen Gehalt an entsprechenden Benzaldehyden von typischerweise weniger als 50 % vorzugsweise weniger als 70 % der ursprünglichen Menge auf.

Der Vorteil der Erfindung liegt in der effizienten Herstellung von hochreinen Verbindungen der Formel (I), die so frei von Nebenkomponenten sind dass sie insbesondere in Metallierungsreaktionen wie Gringnardreaktionen eingesetzt werden können.

Von der Erfindung umfasst ist daher die anschließende Umsetzung der erfindungsgemäß hergestellten Verbindungen der Formel (I) in Metallierungsreaktionen sowie ein Verfahren zur Herstellung von Verbindungen der Formel (V)

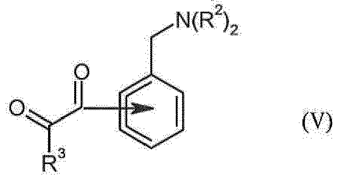

In der die Reste R² die oben genannte Bedeutung einschließlich ihrer Vorzugsbereiche besitzt und R³ für C₁-C₈-Alkyl, C₁-C₈-Alkoxy, C₆-C₁₂-Aryl oder C₇-C₁₃-Arylalkyl steht, dadurch gekennzeichnet, dass die erfindungsgemäß herstellten Verbindungen der Formel (I) zunächst mit seinem Alkali- oder Erdalkalimetall oder einer Verbindung des Typs M(C₁-C₆-alkyl) wobei M für ein Alkalimetall steht und anschließend mit Verbindungen der Formel (VI) umgesetzt werden 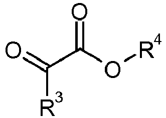 in der der Reste R³ die für Formel (V) genannte Bedeutung besitzt und
- R⁴: für C₁-C₈-Alkyl, C₆-C₁₂-Aryl oder C₇-C₁₃-Arylalkyl steht.

Vorzugsweise erfolgt die Umsetzung der Verbindungen der Formel (I) zunächst mit Magnesium dann mit Oxalsäuredi(C₁-C₈-alkyl)estern.

### Beispiele

### Beispiel 1:

114,4 Gewichtsteile o-Chlorbenzylchlorid 99,7%ig und 176 Gewichtsteile Wasser wurden in einem Autoklav vorgelegt. Dann kondensierte man 156,8 Gewichtsteile Dimethylamin ein. Man erwärmte unter Rühren auf 140°C und rührte 30 min bei dieser Temperatur nach. Es stellte sich ein Druck von ca. 1,5 MPa ein. Danach ließ man auf Raumtemperatur abkühlen und entspannte. Es wurde noch 30 min mit Stickstoff ausgeblasen, um überschüssiges Dimethylamin zu entfernen. Nach Überführung in einen Laborreaktor wurde unter Rühren für 3 h auf 90°C erhitzt, wobei weiteres Dimethylamin ausgetrieben wurde. Bei derselben Temperatur setzte man 117,4 Gewichtsteile 50 Gew.-%ige Natronlauge zu, um die organischen Basen freizusetzen. Man rührte 1 h bei 90°C, ließ absitzen und trennte die Phasen.

Die organische Phase wurde im Vakuum bei 14 mbar über eine Kolonne destilliert. Man trennte einen wasserhaltigen Vorlauf ab, der erneut für die Umsetzung gemäß diesem Beispiel verwendet wurde.

| | | | |
|---|---|---|---|
| Vorlauf: | Kp = 84°C | 24,9 Gewichtsteile | Reinheit: 99,3% |
| Hauptlauf: | Kp = 84°C | 87,7 Gewichtsteile | Reinheit: 99,2% |

Der Hauptlauf enthielt laut HPLC keine detektierbaren Anteile an Bis-(2-chlorbenzyl)-dimethylammoniumchlorid und 2-Chlorbenzalchlorid.

Die Summe der 2 Fraktionen entsprach einer chemischen Ausbeute von 93,0 % d.Th..

### Beispiel 2:

Analog zu Beispiel 1 wurden 76,24 Gewichtsteile o-Chlorbenzylchlorid und 117,4 Gewichtsteile Wasser mit 228,2 Gewichtsteilen Dimethylamin bei 110°C umgesetzt.

Nach wässriger Aufarbeitung wie in Beispiel 1 und Destillation bei 14 mbar erhielt man folgende Fraktionen:

| | | | |
|---|---|---|---|
| Vorlauf: | Kp = 84°C | 0,76 Gewichtsteile | Reinheit: 99,0% |
| Hauptlauf: | Kp = 84°C | 73,3 Gewichtsteile | Reinheit: 99,7% |

Der Hauptlauf enthielt laut HPLC keine detektierbaren Anteile an Bis-(2-chlorbenzyl)-dimethylammoniumchlorid und 2-Chlorbenzalchlorid.

Die Summe der 2 Fraktionen entsprach einer chemischen Ausbeute von 92,2 % d.Th..

### Beispiel 3:

Analog zu Beispiel 1 wurden 305 Gewichtsteile o-Chlorbenzylchlorid und 438,1 Gewichtsteile Wasser mit 876,3 Gewichtsteilen Dimethylamin bei 140°C umgesetzt.

Nach wässriger Aufarbeitung wie in Beispiel 1 und Destillation bei 50 mbar erhielt man folgende Fraktionen:

| | | | |
|---|---|---|---|
| Vorlauf: | Kp = 114°C | 9,6 Gewichtsteile | Reinheit: 99,6 % |
| Hauptlauf: | Kp = 117°C | 259,1 Gewichtsteile | Reinheit: 99,9 % |
| Sumpf: | | 38,3 Gewichtsteile | Reinheit: 96,7 % |

Der Hauptlauf enthielt laut HPLC keine detektierbaren Anteile an Bis-(2-chlorbenzyl)-dimethylammoniumchlorid und 2-Chlorbenzalchlorid.

Die Summe der 2 Fraktionen entsprach einer chemischen Ausbeute von 95,4 % d.Th..

### Beispiel 4:

Analog zu Beispiel 1 wurden 76,24 Gewichtsteile o-Chlorbenzylchlorid und 117,4 Gewichtsteile Wasser mit 228,2 Gewichtsteilen Dimethylamin bei 170°C umgesetzt.

Nach wässriger Aufarbeitung wie in Beispiel 1 und Destillation bei 14 mbar erhielt man folgende Fraktionen:

| | | | |
|---|---|---|---|
| Vorlauf: | Kp = 84°C | 1,6 Gewichtsteile | Reinheit: 99,1% |
| Hauptlauf: | Kp = 84°C | 71,5 Gewichtsteile | Reinheit: 99,2% |
| Sumpf: | | 2,0 Gewichtsteile | Reinheit: 88,9% |

Der Hauptlauf enthielt laut HPLC keine detektierbaren Anteile an Bis-(2-chlorbenzyl)-dimethylammoniumchlorid und 2-Chlorbenzalchlorid.

Die Summe der 2 Fraktionen entsprach einer chemischen Ausbeute von 92,8 % d.Th..

### Beispiel 5:

Der Versuch aus Beispiel 3 wurde wiederholt. Vor der Destillation wurden 33,4 Gewichtsteile des Destillationsrückstandes aus Beispiel 3 zugesetzt.

Nach Destillation bei 50 mbar erhält man folgende Fraktionen:

| | | | |
|---|---|---|---|
| Vorlauf: | Kp = 108-116°C | 27,9 Gewichtsteile | Reinheit: 97,6% |
| Hauptlauf: | Kp = 116°C | 299,7 Gewichtsteile | Reinheit: 99,2% |
| Sumpf: | | 15,6 Gewichtsteile | Reinheit: 86,2% |

Der Hauptlauf enthielt laut HPLC keine detektierbaren Anteile an Bis-(2-chlorbenzyl)-dimethylammoniumchlorid und 2-Chlorbenzalchlorid.

### Beispiel 6:

Einem Reaktionsrohr von 4,80 m Länge und 2 mm Durchmesser, das mit einem Druckhalteventil versehen war, wurden folgende Eduktströme über Pumpen zugeführt:
76,33 g/h o-Chlorbenzylchlorid
220,0 g/h Dimethylamin
110,0 g/h Wasser

Dabei wurden zuerst die Ströme Dimethylamin und Wasser vereinigt und auf 130°C vorgewärmt. Dazu wurde dann über eine Mischkammer der Strom von o-Chlorbenzylchlorid zugeführt. Im Reaktionsrohr stellte sich eine Temperatur von etwa 140°C ein. Nach Durchlaufen der Reaktionsstrecke wurde abgekühlt und entspannt. Die Aufarbeitung analog Beispiel 1 lieferte dann das Zielprodukt (2-Chlorbenzyl)-dimethylamin in einer Ausbeute und Reinheit wie in Beispiel 3.

### Beispiel 7:

144 Gewichtsteile eines (2-Chlorbenzyl)-dimethylamins, das nach Beispiel 1 hergestellt wurde und noch 0,1 Gew% o-Chlorbenzaldehyd enthielt, wurde auf 60°C erwärmt. Dann wurden 0,27 Gewichtsteile Natriumborhydrid zugegeben und 3 h bei 60°C nachgerührt.

Eine HPLC-Analyse ergab, dass der Gehalt an o-Chlorbenzaldehyd auf 0,017 Gew-% gesunken war.

Das Produkt wurde dann bei 50 mbar destilliert. Man trennte einen wasserhaltigen Vorlauf ab, der im nächsten Ansatz wieder eingesetzt wurde.
Vorlauf: Kp = 106°C, 14,4 Gewichtsteile, Reinheit: 99,4%, Gehalt an o-Chlorbenzaldehyd: 0,043%
Hauptlauf: Kp = 116°C, 93 Gewichtsteile, Reinheit: 99,0%, Gehalt an o-Chlorbenzaldehyd: 0,024%
Sumpf:23,3 Gewichtsteile, Reinheit: 94,3%, Gehalt an o-Chlorbenzaldehyd: 0,034%

### Vergleichsbeispiel 8:

Die Destillation von 317 Gewichtsteilen eines (2-Chlorbenzyl)-dimethylamins, das nach Beispiel 1 hergestellt wurde, wurde ohne Vorbehandlung gemäß Beispiel 7 bei 50 mbar durchgeführt. Man erhielt folgende Fraktionen:
Vorlauf: Kp = 114°C, 9,6 Gewichtsteile, Reinheit: 99,5 %, Gehalt an o-Chlorbenzaldehyd: 0,43 %
Hauptlauf: Kp = 117°C, 259,1 Gewichtsteile, Reinheit: 99,9 %, Gehalt an o-Chlorbenzaldehyd: 0,097°
Sumpf: 38,3 Gewichtsteile, Reinheit: 96,7%, Gehalt an o-Chlorbenzaldehyd: 0,23%

### Beispiel 9:

Analog zu Beispiel 6 wurde die Umsetzung in einem Reaktionsrohr von 42 m Länge und 0,7 mm Durchmesser durchgeführt. Folgende Eduktströme wurden eingesetzt:
123 g/h o-Chorbenzylchorid
240 g/h Dimethylamin und
154 g/h Wasser.

Wasser und Dimethylamin wurden wie in Beispiel 6 vor der Mischkammer vereinigt und auf 120°C vorgewärmt. Das Reaktionsrohr wurde 29 h bei 130°C betrieben. Nach Entspannung des Dimethylamins wurde das Reaktionsgemisch in einen 25 1 Rührapparat überführt und langsam auf 90°C erwärmt, um weiteres Dimethylamin auszugasen. Es wurde 1 h bei 90°C nachgerührt. Dann wurde über 3,5 h bei 90°C 4998 g 50 %ige Natronlauge zudosiert, wobei weiteres Dimethylamin ausgaste. Es wurde 1 h bei 90°C nachgerührt. Bei dieser Temperatur wurden nur die Phasen getrennt, wobei 3647 g organische Phase anfielen. 1600 g der oberen organischen Phase wurden durch Vakuumdestillation bei 200 mbar aufgearbeitet. Man erhielt folgende Fraktionen:

| | | | |
|---|---|---|---|
| Vorlauf: | Kp = 59-151°C | 13,6 g | Reinheit: 75,6 % |
| Hauptlauf: | Kp = 152-153°C | 1406 g | Reinheit: 99,6 % |
| Sumpf: | | 138,8 g | Reinheit: 90,1 % |

Die Summe dieser Fraktionen entspricht einer chemischen Ausbeute von 93,8 %

## Patentansprüche

1. Verfahren zur Herstellung von Verbindungen der Formel (I) 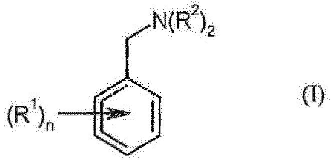 in der
n für 0, 1, 2 oder 3, bevorzugt für I oder 2, besonders bevorzugt für 1 steht
die Reste R¹ jeweils unabhängig voneinander für ein Halogenatom stehen, dass an den aromatischen Kern gebunden ist, vorzugsweise für Chlor oder Brom, noch weiter bevorzugt für Chlor
die Reste R² jeweils unabhängig voneinander für C₁-C₈-Akyl, C₆-C₁₂-Aryl oder C₇-C₁₃-Arylalkyl oder die beiden Reste R² zusammen für einen geradkettigen oder verzweigten C₃-C₁₂-Alkylen-Rest stehen, der gegebenenfalls durch ein oder mehrere Sauerstoffatome unterbrochen sein kann
durch Umsetzung von Verbindungen der Formel (II) 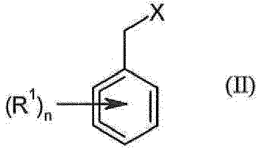 mit Verbindungen der Formel (III)
HN(R²)₂ (III)
wobei in den Formeln (I) und (II) die Reste R¹, R² und R³ die unter der Formel (I) genannte Bedeutung besitzen und
X für Chlor, Brom, Iod, -OSO₂-(C₁-C₆-Alkyl) oder -OSO₂-(C₁-C₆-Perfluoralkyl), bevorzugt für Chlor oder Brom, ganz besonders bevorzugt für Chlor steht
wobei das Verfahren **dadurch gekennzeichnet ist, dass**
• das molare Verhältnis von Verbindungen der Formel (III) zu Verbindungen der Formel (II) 3:1 oder größer ist, vorzugsweise 3:1 bis 50:1, besonders bevorzugt 3:1 bis 20:1 und ganz besonders bevorzugt 5:1 bis 20:1 sowie in einer anderen Ausführungsform 5:1 oder größer, vorzugsweise 5:1 bis 50:1, besonders bevorzugt 5:1 bis 20:1 und ganz besonders bevorzugt 8:1 bis 14:1
• organische Lösungsmittel in einer Menge von 10 Gew.-% oder weniger, vorzugsweise 5 Gew.-% oder weniger, besonders bevorzugt 0 bis 2 Gew.-% bezogen auf die Summe an eingesetzten Verbindungen der Formel (II) und (III) eingesetzt werden und ganz besonders bevorzugt kein organisches Lösungsmittel eingesetzt wird und
• Wasser eingesetzt wird, vorzugsweise mehr als 10 Gew. %, besonders bevorzugt mehr als 30 Gew.-%, ganz besonders bevorzugt mehr als 50 Gew.-% und insbesondere bevorzugt mehr als 100 Gew.-% Wasser, insbesondere ganz bevorzugt 100 bis 1000 Gew.-% bezogen auf die eingesetzte Verbindung der Formel (II),
• bei einer Reaktionstemperatur von 90-180°C.

2. Verfahren nach Anspruch 1, **dadurch gekennzeichnet, dass** 2-Chlor-N,N-dimethylbenzylamin durch Umsetzung von 2-Chlor-benzylchlorid mit Dimethylamin hergestellt wird.

3. Verfahren nach Anspruch 1 oder 2, **dadurch gekennzeichnet, dass** die Reaktionstemperatur 130 bis 150°C liegt.

4. Verfahren nach einem der Ansprüche 1 bis 3, **dadurch gekennzeichnet, dass** der Reaktionsdruck 2 bis 300 bar, vorzugsweise 4 bis 50 bar und besonders bevorzugt 5 bis 35 bar beträgt.

5. Verfahren nach einem der Ansprüche 1 bis 4, **dadurch gekennzeichnet, dass** es kontinuierlich durchgeführt wird.

6. Verfahren zur Herstellung von Verbindungen der Formel (I) gemäß Anspruch 1, mit einem Gehalt an Verbindungen der Formel (IV) 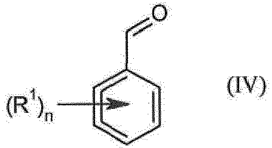 in der R¹, die in Anspruch 1 genannte Bedeutung besitzt
von vorzugsweise 0,050 bis 2,0 Gew.-%, besonders bevorzugt 0,1 bis 1,0 Gew.-%, **dadurch gekennzeichnet, dass** sie mit einem Alkalimetallborhydrid oder einem Alkalimetallaluminiumhydrid umgesetzt werden und gegebenenfalls aber vorzugsweise anschließend destilliert werden.

7. Verfahren nach Anspruch 6, **dadurch gekennzeichnet, dass** 2-Chlor-N,N-dimethylbenzylamin mit einem Gehalt an 2-Chlorbenzaldehyd eingesetzt wird.

8. Verfahren nach Anspruch 6 oder 7, **dadurch gekennzeichnet, dass** Natriumborhydrid eingesetzt wird.

9. Verfahren nach einem der Ansprüche 6 bis 8, **dadurch gekennzeichnet, dass** es im Anschluss an ein Verfahren gemäß den Ansprüchen 1 bis 5 durchgeführt wurde.

10. Verfahren nach einem der Ansprüche 1 bis 9, **dadurch gekennzeichnet, dass** Verbindungen der Formel (I) anschließend in Metallierungsreaktionen eingesetzt werden.

11. Verfahren nach Anspruch 10, **dadurch gekennzeichnet, dass** die Metalliertingsreaktion eine Grignardreaktion ist.

12. Verfahren gemäß einem der Ansprüche 1 bis 9, **dadurch gekennzeichnet, dass** die Verbindung der Formel (1) mit einem Alkali- oder Erdalkalimetall oder einer Verbindung des Typs M(C₁-C₆-Alkyl) wobei M für ein Alkalimetall steht, und anschließend mit Verbindungen der Formel (VI) 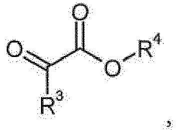 in der der Reste R³ die für Formel (V) genannte Bedeutung besitzt und
R⁴ für C₁-C₈-Alkyl, C₆-C₁₂-Aryl oder C₇-C₁₃-Arylalkyl steht, umgesetzt wird zur Herstellung der Verbindung der Formel (V)

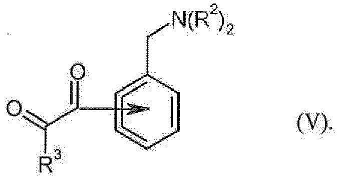

## Claims

1. Method for preparing compounds of the formula (I) 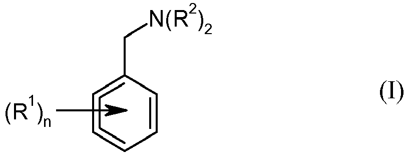 in which
n is 0, 1, 2 or 3, preferably 1 or 2, particularly preferably 1
the residues R¹ are each independently a halogen atom, preferably chlorine or bromine, more preferably chlorine, which is bonded to the aromatic ring
the residues R² are each independently C₁-C₈-alkyl, C₆-C₁₂-aryl or C₇-C₁₃-arylalkyl or both residues R² together are a straight-chain or branched C₃-C₁₂-alkylene residue, which may optionally be interspersed by one or more oxygen atoms
by reacting compounds of the formula (II) 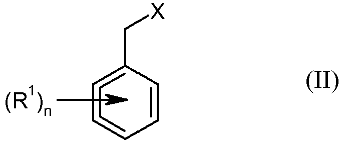 with compounds of the formula (III)
HN(R²)₂ (III)
where the residues R¹, R² and R³ in the formulae (I) and (II) have the meanings stated under the formula (I) and
X is chlorine, bromine, iodine, -OSO₂-(C₁-C₆-alkyl) or -OSO₂-(C₁-C₆-perfluoroalkyl), preferably chlorine or bromine, especially preferably chlorine
wherein the method is **characterized in that**
• the molar ratio of compounds of the formula (III) to compounds of the formula (II) is 3:1 or greater, preferably 3:1 to 50:1, particularly preferably 3:1 to 20:1 and especially preferably 5:1 to 20:1 and also in another embodiment 5:1 or greater, preferably 5:1 to 50:1, particularly preferably 5:1 to 20:1 and especially preferably 8:1 to 14:1
• organic solvent is used in an amount of 10% by weight or less, preferably 5% by weight or less, particularly preferably 0 to 2% by weight, based on the sum of compounds of the formulae (II) and (III) used and especially preferably no organic solvent is used and
• water is used, preferably more than 10% by weight, particularly preferably more than 30% by weight, especially preferably more than 50% by weight and particularly preferably more than 100% by weight of water, especially preferably 100 to 1000% by weight, based on the compound of the formula (II) used,
• at a reaction temperature of 90 - 180°C.

2. Method according to Claim 1, **characterized in that** 2-chloro-N,N-dimethylbenzylamine is prepared by reacting 2-chlorobenzyl chloride with dimethylamine.

3. Method according to Claim 1 or 2, **characterized in that** the reaction temperature is 130 to 150°C.

4. Method according to any of Claims 1 to 3, **characterized in that** the reaction pressure is 2 to 300 bar, preferably 4 to 50 bar and particularly preferably 5 to 35 bar.

5. Method according to any of Claims 1 to 4, **characterized in that** said method is carried out continuously.

6. Method for preparing compounds of the formula (I) according to Claim 1, having a content of compounds of the formula (IV) 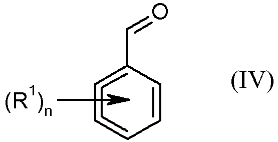 in which R¹ has the meaning stated in Claim 1
of preferably 0.050 to 2.0% by weight, particularly preferably 0.1 to 1.0% by weight, **characterized in that** said compounds are reacted with an alkali metal borohydride or an alkali metal aluminum hydride and are optionally although preferably subsequently distilled.

7. Method according to Claim 6, **characterized in that** 2-chloro-N,N-dimethylbenzylamine is used containing 2-chlorobenzaldehyde.

8. Method according to Claim 6 or 7, **characterized in that** sodium borohydride is used.

9. Method according to any of Claims 6 to 8, **characterized in that** said method was carried out subsequent to a method according to Claims 1 to 5.

10. Method according to any of Claims 1 to 9, **characterized in that** compounds of the formula (I) are subsequently used in metalation reactions.

11. Method according to Claim 10, **characterized in that** the metalation reaction is a Grignard reaction.

12. Method according to any of Claims 1 to 9, **characterized in that** the compound of the formula (I) is reacted with an alkali metal or alkaline earth metal or a compound of the type M(C₁-C₆-alkyl) where M is an alkali metal, and subsequently with compounds of the formula (VI) 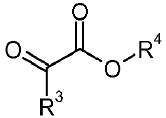 in which the residue R³ has the meaning stated for formula (V) and
R⁴ is C₁-C₈-alkyl, C₆-C₁₂-aryl or C₇-C₁₃-arylalkyl, for preparing the compound of the formula (V)

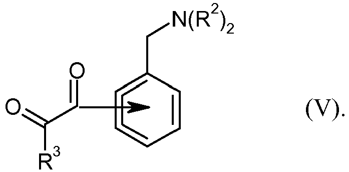

## Revendications

1. Procédé pour la préparation de composés de formule (I) 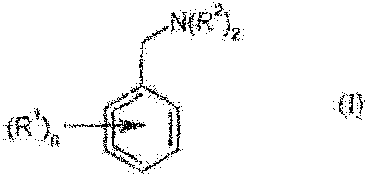 dans laquelle
n représente 0, 1, 2 ou 3, de préférence 1 ou 2, de façon particulièrement préférée 1
les radicaux R¹ représentent chacun indépendamment un atome d'halogène qui est lié au noyau aromatique, de préférence un atome de chlore ou de brome, de façon encore plus particulièrement préférée un atome de chlore
les radicaux R² représentent chacun, indépendamment l'un de l'autre, un groupe alkyle en C₁-C₈, aryle en C₆-C₁₂ ou arylalkyle en C₇-C₁₃, ou les deux radicaux R² représentent ensemble un radical alkylène en C₃-C₁₂ à chaîne droite ou ramifié, qui peut éventuellement être interrompu par un ou plusieurs atomes d'oxygène,
par mise en réaction de composés de formule (II) 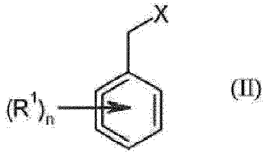 avec des composés de formule (III)
HN(R²)₂ (III)
dans les formules (I) et (II) les radicaux R¹, R² et R³ ayant la signification donnée sous la formule (I) et
X représentant un atome de chlore, de brome, d'iode, un groupe -OSO₂-(alkyle(C₁-C₆)) ou -OSO₂-(perfluoroalkyle(C₁-C₆)), de préférence un atome de chlore ou de brome, de façon tout particulièrement préférée un atome de chlore,
le procédé étant **caractérisé en ce que**
• le rapport molaire des composés de formule (III) aux composés de formule (II) est égal ou supérieur à 3:1, de préférence vaut de 3:1 à 50:1, de façon particulièrement préférée de 3:1 à 20:1 et de façon tout particulièrement préférée de 5:1 à 20:1, ainsi que dans un autre mode de réalisation est égal ou supérieur à 5:1, de préférence vaut de 5:1 à 50:1, de façon particulièrement préférée de 5:1 à 20:1 et de façon tout particulièrement préférée de 8:1 à 14:1.
• on utilise des solvants organiques en une quantité de 10 % en poids ou moins, de préférence de 5 % en poids ou moins, de façon particulièrement préférée de 0 à 2 % en poids, par rapport à la somme des composés de formules (II) et (III) utilisés, et de façon tout particulièrement préférée on n'utilise pas de solvant organique et
• on utilise de l'eau à raison de préférence de plus de 10 % en poids, de façon particulièrement préférée de plus de 30 % en poids, de façon tout particulièrement préférée de plus de 50 % en poids et de façon plus particulièrement préférée de plus de 100 % en poids d'eau, en particulier de façon tout particulièrement préférée de 100 à 1 000 % en poids par rapport au composé de formule (II) utilisé,
• à une température de 90 à 180 °C.

2. Procédé selon la revendication 1, **caractérisé en ce qu'**on prépare de la 2-chloro-N,N-diméthylbenzylamine en faisant réagir du chlorure de 2-chlorobenzyle avec de la diméthylamine.

3. Procédé selon la revendication 1 ou 2, **caractérisé en ce que** la température de réaction vaut de 130 à 150 °C.

4. Procédé selon l'une quelconque des revendications 1 à 3, **caractérisé en ce que** la pression de la réaction vaut de 2 à 300 bars, de préférence de 4 à 50 bars et de façon particulièrement préférée de 5 à 35 bars.

5. Procédé selon l'une quelconque des revendications 1 à 4, **caractérisé en ce qu'**il est effectué en continu.

6. Procédé pour la préparation de composés de formule (I) selon la revendication 1, ayant une teneur en composés de formule (IV) 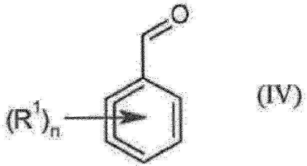 dans laquelle R¹ a la signification indiquée dans la revendication 1,
de préférence de 0,050 à 2,0 % en poids, de façon particulièrement préférée de 0,1 à 1,0 % en poids,
**caractérisé en ce qu'**on les fait réagir avec un borohydrure de métal alcalin ou un hydrure d'aluminium et de métal alcalin et éventuellement mais de préférence ensuite on les distille.

7. Procédé selon la revendication 6, **caractérisé en ce qu'**on utilise de la 2-chloro-N,N-diméthylbenzylamine ayant une teneur en 2-chlorobenzaldéhyde.

8. Procédé selon la revendication 6 ou 7, **caractérisé en ce qu'**on utilise du borohydrure de sodium.

9. Procédé selon l'une quelconque des revendications 6 à 8, **caractérisé en ce qu'**il a été effectué à la suite d'un procédé selon les revendications 1 à 5.

10. Procédé selon l'une quelconque des revendications 1 à 9, **caractérisé en ce que** les composés de formule (I) sont ensuite utilisés dans des réactions de métallation.

11. Procédé selon la revendication 10, **caractérisé en ce que** la réaction de métallation est une réaction de Grignard.

12. Procédé selon l'une quelconque des revendications 1 à 9, **caractérisé en ce qu'**on fait réagir le composé de formule (I) avec un métal alcalin ou alcalino-terreux ou un composé du type M(alkyle(C₁-C₆)), M représentant un métal alcalin, et ensuite avec des composés de formule (VI) 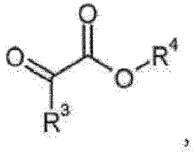 dans laquelle les radicaux R³ ont la signification indiquée pour la formule (V) et
R⁴ représente un groupe alkyle en C₁-C₈, aryle en C₆-C₁₂ ou arylalkyle en C₇-C₁₃,
pour la préparation du composé de formule (V)
